# EUROPEAN PATENT SPECIFICATION

(11) **EP 3 792 562 B1**
(45) Date of publication and mention of the grant of the patent: **12.07.2023**
(21) Application number: 19815116.9
(22) Date of filing: 07.06.2019
(51) Int. Cl.: F24F 7/06, A61L 9/16, F24F 13/06, B08B 5/02, F24F 3/163, F24F 9/00, F24F 8/20, F24F 8/50

(54) **DUST REMOVAL DEVICE AND DUST REMOVAL SYSTEM**
STAUBENTFERNUNGSVORRICHTUNG UND STAUBENTFERNUNGSSYSTEM
DISPOSITIF DE DÉPOUSSIÉRAGE ET SYSTÈME DE DÉPOUSSIÉRAGE

(30) Priority: 08.06.2018 JP 2018110176
(43) Date of publication of application: 17.03.2021
(73) Proprietor: DAIKIN INDUSTRIES, LTD., Osaka-Shi, Osaka 530-0001 (JP)
(72) Inventor: SUZUMURA, Kei, Osaka-shi, Osaka 530-8323 (JP); YABU, Tomohiro, Osaka-shi, Osaka 530-8323 (JP)
(74) Representative: Goddar, Heinz J.
(86) International application number: PCT/JP2019/022718
(87) International publication number: WO 2019/235612

(56) References cited:
- JP-A- H04 313 632
- JP-A- H04 313 632
- JP-A- H05 141 732
- JP-A- H09 273 783
- JP-A- 2007 255 778
- JP-A- 2008 075 349
- JP-A- 2016 109 414
- JP-B2- 5 464 726
- KR-A- 20140 135 074
- US-A1- 2016 150 925

## Description

### Technical Field

The present invention relates to a dust removal device and a dust removal system.

### Background Art

There is a known air shower device that blows air with respect to a subject (a person that enters a room) through an air nozzle to beat powder dust out of the subject (refer to, for example, PTL 1).

### Citation List

### Patent Literature

PTL 1: Japanese Patent No. 5464726
PTL 2: US 2016/150925 A1
PTL 3: JP H04 31 3632 A
PTL 4: KR 2014 0135074 A
PTL 5: JP 2007 255778 A

PTL 2 discloses separating and removing dust attached to a person from the person in a room after the person enters the room. PTL 2 discloses a dust removal device comprising the features of the preamble of claim 1.

PTL 3 to PTL 5 disclose removing dust attached to a person from the person in an air shower room after the person enters the air shower room.

### Summary of Invention

### Technical Problem

Such an air shower device known in the art, however, blows air to the whole body of a subject and thus has a likelihood of making a subject for which air is blown onto the face thereof feel discomfort.

An object of the present invention is to provide an idea regarding the blow-out location of air to suppress a subject from feeling discomfort.

### Solution to Problem

A first aspect of the present invention relates to a dust removal device according to claim 1.

In the first aspect, the blow-out location of air is changed in accordance with the height of the subject (T), thereby causing the air to be blown toward the subject (T) from the neck down. Consequently, the air can be suppressed from being blown onto the face of the subject (T).

Further in the first aspect of the present invention the blow-out location changing part (23) is configured to blow air toward the subject (T) in a direction from an indoor space (S1) to an outdoor space (S2).

In this aspect, the air is blown toward the subject (T) in the direction from the indoor space (S1) to the outdoor space (S2). Consequently, after the dust adhering to the subject (T) is removed, the dust can be discharged along the flow of the air to the outdoor space (S2).

A second aspect of the present invention is the first aspect in which the blow-out location changing part (23) is configured to blow air toward the subject (T) downwardly from above.

In the second aspect, the air is blown toward the subject (T) downwardly from above. Consequently, after the dust adhering to the subject (T) is removed, the dust can be suppressed from floating and adhering again to the subject (T) .

Further in the first aspect of the present invention the dust removal device has blow-out ports (22) provided such that air is blown in a lateral direction that crosses an entrance direction in which the subject (T) moves from the outdoor space (S2) toward the indoor space (S1), and in which the blow-out port (22) that opens at a near side in the entrance direction is positioned higher than the blow-out port (22) that opens at a far side in the entrance direction.

In this aspect, the blow-out port (22) that opens at the near side in the entrance direction is positioned higher than the blow-out port (22) that opens at the far side in the entrance direction. Specifically, when the subject (T) moves in the entrance direction, the dust on the upper body of the subject (T) is first removed. Thereafter, even when the removed dust adheres again to the lower body of the subject (T), the dust on the lower body of the subject (T) can be removed by the air blown through the blow-out port (22) at the far side.

A third aspect of the present invention is any one of the first to second aspects in which the dust removal device includes an addition part (12) that adds a useful component to air that is blown by the fan (25).

In the third aspect, the useful component is added to the air that is blown by the fan (25). Consequently, for example, a deodorizer, a sterilizing agent, a fragrance agent, and the like can be added as the useful component.

A fourth aspect of the present invention is intended for a dust removal system including the dust removal device (10) according to any one of the first to third aspects, the dust removal device (10) being for the subject (T), and a dust removal device (40) that is for an article (B) and that blows air toward the article (B) and remove dust.

In the fourth aspect, the dust removal device (10) for the subject (T) and the dust removal device (40) for the article (B) are included. Consequently, the dust adhering to the subject (T) and the dust adhering to the article (B) can be removed by different dust removal devices (10 and 40).

### Brief Description of Drawings

[Fig. 1] Fig. 1 is a perspective view of a configuration of a dust removal device according to Embodiment 1.
[Fig. 2] Fig. 2 is a planar sectional view of a state in which air is blown in a direction from an indoor space to an outdoor space.
[Fig. 3] Fig. 3 is a side view of a state in which air is blown toward a subject from the neck down in accordance with the height of the subject.
[Fig. 4] Fig. 4 is a back view of a state in which air is blown to a subject downwardly from above.
[Fig. 5] Fig. 5 is a side view of a state in which air is blown toward another subject from the neck down in accordance with the height of the other subject.
[Fig. 6] Fig. 6 is a side view of a configuration in which a blow-out location is changeable by switching the opening and closing operations of an opening-closing damper in a dust removal device according to Modification 1.
[Fig. 7] Fig. 7 is a side view of a configuration in which a blow-out location is changeable by switching the operation of a plurality of fans in a dust removal device according to Modification 2.
[Fig. 8] Fig. 8 is a plan view of a configuration of a dust removal system according to Embodiment 2, the dust removal system including a dust removal device for a subject and a dust removal device for an article.

### Description of Embodiments

### <<Embodiment 1>>

Embodiment 1 will be described.

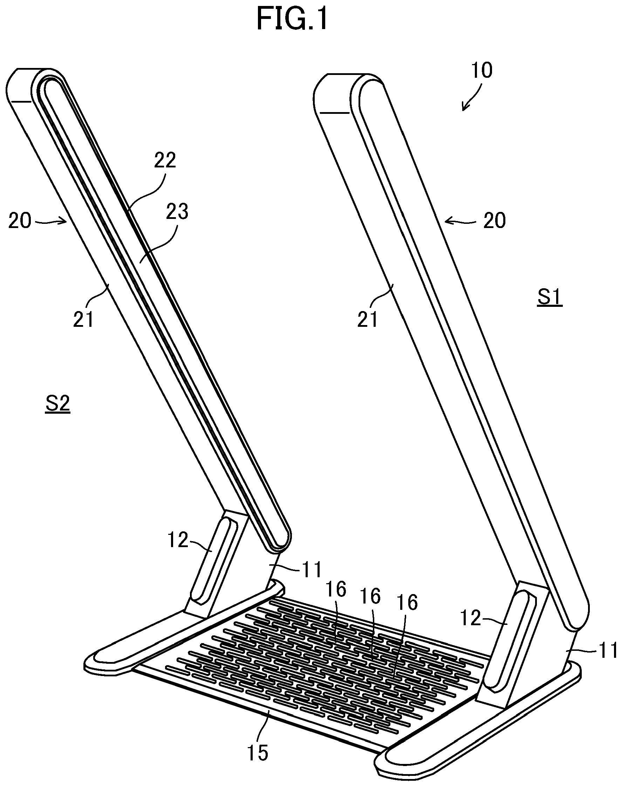

As illustrated in Fig. 1, a dust removal device (10) includes a left-right pair of blower gates (20), left and right support legs (11) that support lower end portions of the blower gates (20), and a floor plate (15) disposed between the left and right support legs (11).

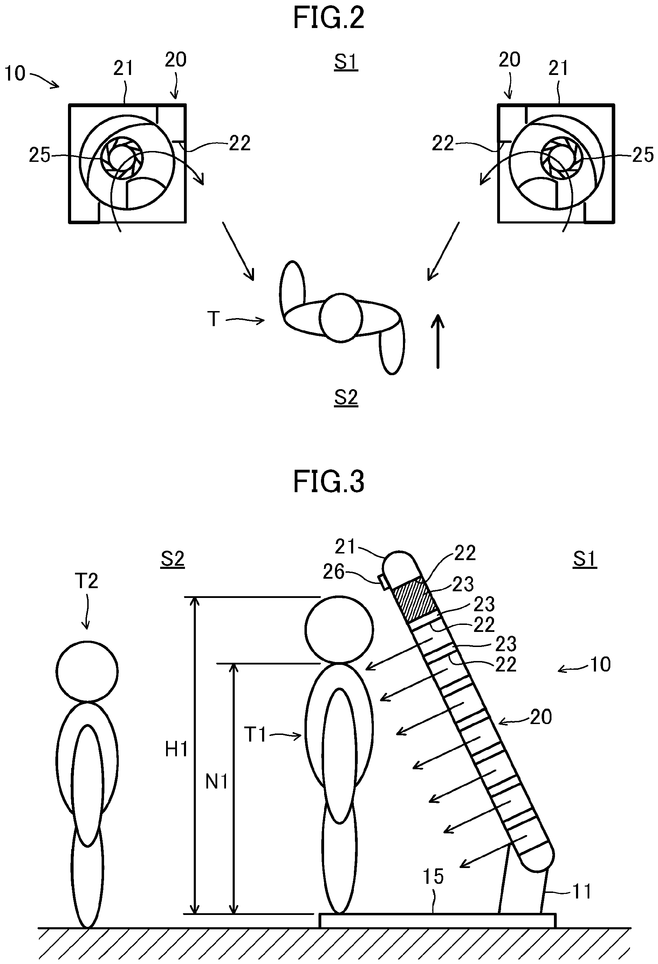

As illustrated in Fig. 2 and Fig. 3, the blower gates (20) each include a gate body (21) with a cross-flow type fan (25) disposed in the inner portion thereof, and a plurality of flaps (23) (blow-out location changing parts) capable of opening and closing the plurality of blow-out ports (22) formed in the gate body (21).

The blower gates (20) are installed at a boundary location between an indoor space (S1) and an outdoor space (S2). The blow-out ports (22) are formed such that air is blown in a lateral direction that crosses an entrance direction in which a subject (T) moves from the outdoor space (S2) toward the indoor space (S1). In other words, the blow-out ports (22) are formed in faces of the left and right blower gates (20), the faces facing each other.

The blower gates (20) each stand in an orientation that is inclined obliquely upward from the indoor space (S1) toward the outdoor space (S2). Consequently, the blow-out ports (22) that open at the near side in the entrance direction are positioned higher than the blow-out ports (22) that open at the far side in the entrance direction.

The support legs (11) support the blower gates (20) so as to be in the above-described inclined orientation. An addition cartridge (12) (an addition part) that adds a useful component to the air blown by the fans (25) is detachably mounted on each of the support legs (11) (refer to Fig. 1).

The useful component contained in the addition cartridge (12) is, for example, a deodorizer, a sterilizing agent, a fragrance agent, and the like. The inner portion of each support leg (11) and the inner portion of the gate body (21) corresponding thereto are in communication with each other. When the fans (25) of the blower gates (20) are driven to rotate, the useful component of the addition cartridges (12) is sent into the inner portions of the gate bodies (21) and blown through the blow-out ports (22) after stirred with air.

A plurality of suction ports (16) are formed at the upper surface of the floor plate (15). The suction ports (16) are in communication with a space of the inner portion of the floor plate (15). In the space of the inner portion of the floor plate (15), a suction mechanism (not illustrated) sucks air.

The dust adhering to the subject (T) is sucked through the suction ports (16) after removed by the air blown from the blower gates (20) toward the subject (T) and is collected in the floor plate (15). Consequently, the dust can be suppressed from floating and adhering again to the subject (T).

When air is blown toward the whole body of the subject (T), the air is also blown onto the face of the subject (T) and has a likelihood of making the subject (T) feel discomfort or disarranging a set hair style thereof.

Thus, in the present embodiment, the blow-out location is changeable in accordance with the height of the subject (T) such that air is blown toward the subject (T) from the neck down.

Specifically, as illustrated in Fig. 3, the blower gates (20) are provided with a detection sensor (26) that detects a height (H1) of a subject (T1) that intends to pass between the blower gates (20). A position (N1) of the neck of the subject (T1) is estimated on the basis of a result of the detection by the detection sensor (26). An image of the whole body of the subject (T1) may be obtained by a camera (not illustrated) or the like, and the position (N1) of the neck of the subject (T1) in the image may be extracted.

The blow-out port (22) in a location where air is to be blown to the face of the subject (T1) is closed by the flaps (23), thereby causing the air to be blown to the subject (T1) from the neck down. In the example illustrated in Fig. 3, among eight blow-out ports (22), the first blow-out port (22) from the top is closed by the flaps (23), which are indicated by hatching.

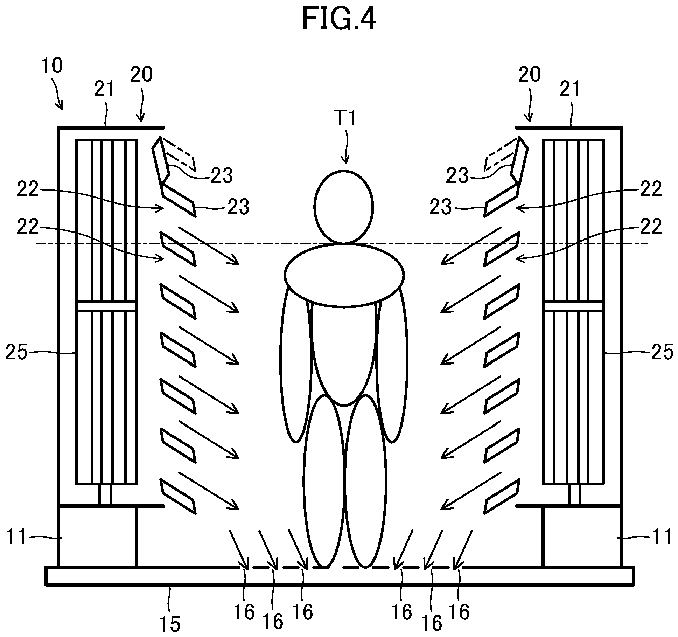

Moreover, as illustrated in Fig. 4, the rotation angle of the flaps (23) is adjusted, thereby causing the air to be blown downwardly from above the subject (T1). Consequently, the dust can be suppressed from floating and adhering again to the subject (T). The removed dust is collected in the inner portion of the floor plate (15) through the suction ports (16) of the floor plate (15).

Meanwhile, dust of pollen and the like largely adheres to the body parts of the subject (T1), and the amount of dust adhering to the parts of the face is small compared with the body parts. Therefore, the influence as a result of the dust that adheres to the parts of the face being taken into the indoor space (S1) is small.

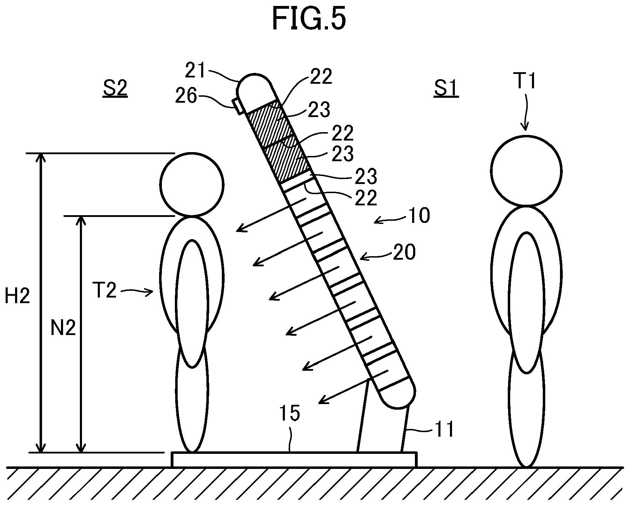

Next, as illustrated in Fig. 5, when a subject (T2) whose height is lower than that of the subject (T1) passes between the blower gates (20), a height (H2) of the subject (T2) is first detected by the detection sensor (26). Then, on the basis of a result of the detection by the detection sensor (26), a position (N2) of the neck of the subject (T2) is estimated.

Then, the blow-out ports (22) in a location where air is to be blown to the face of the subject (T2) are closed by the flaps (23), thereby causing the air to be blown to the subject (T2) from the neck down. In the example illustrated in Fig. 5, among the eight blow-out ports (22), the first and second blow-out ports (22) from the top are closed by the flaps (23), which are indicated by hatching.

### - Effects of Embodiment 1 -

The dust removal device (10) according to the present embodiment blows air toward the subject (T) and removes dust. The dust removal device (10) includes the fans (25) that blow air and the flaps (23) (blow-out location changing parts) that change the blow-out location of the air that is blown by the fans (25) and moves toward the subject (T). The flaps (23) are configured to change the blow-out location in accordance with the height of the subject (T) such that air is blown toward the subject (T) from the neck down.

In the present embodiment, the blow-out location of the air is changed in accordance with the height of the subject (T), thereby causing the air to be blown toward the subject (T) from the neck down. Consequently, the air can be suppressed from being blown onto the face of the subject (T).

Further, in the dust removal device (10) according to the present embodiment, the flaps (23) (blow-out location changing parts) are configured to blow air toward the subject (T) in a direction from the indoor space (S1) to the outdoor space (S2).

In the present embodiment, air is caused to be blown toward the subject (T) in the direction from the indoor space (S1) to the outdoor space (S2). Consequently, after the dust adhering to the subject (T) is removed, the dust can be discharged along the flow of the air to the outdoor space (S2).

In the dust removal device (10) according to the present embodiment, the flaps (23) (blow-out location changing parts) are configured to blow air toward the subject (T) downwardly from above.

In the present embodiment, the air is caused to be blown toward the subject (T) downwardly from above. Consequently, after the dust adhering to the subject (T) is removed, the dust can be suppressed from floating and adhering again to the subject (T).

In the dust removal device (10) according to the present embodiment, the blow-out ports (22) are provided such that air is blown in the lateral direction that crosses the entrance direction in which the subject (T) moves from the outdoor space (S2) toward the indoor space (S1), and the blow-out ports (22) that open at the near side in the entrance direction are positioned higher than the blow-out ports (22) that open at the far side in the entrance direction.

In the present embodiment, the blow-out ports (22) that open at the near side in the entrance direction are positioned higher than the blow-out ports (22) that open at the far side in the entrance direction. Specifically, when the subject (T) moves in the entrance direction, the dust on the upper body of the subject (T) is first removed. Thereafter, even when the removed dust adheres again to the lower body of the subject (T), the dust on the lower body of the subject (T) can be removed by the air blown through the blow-out ports (22) at the far side.

The dust removal device (10) according to the present embodiment also includes the addition cartridges (12) that add the useful component to the air blown by the fans (25).

In the present embodiment, the useful component is added to the air that is blown by the fans (25). Consequently, for example, a deodorizer, a sterilizing agent, a fragrance agent, and the like can be added as the useful component.

### - Modification 1 of Embodiment 1 -

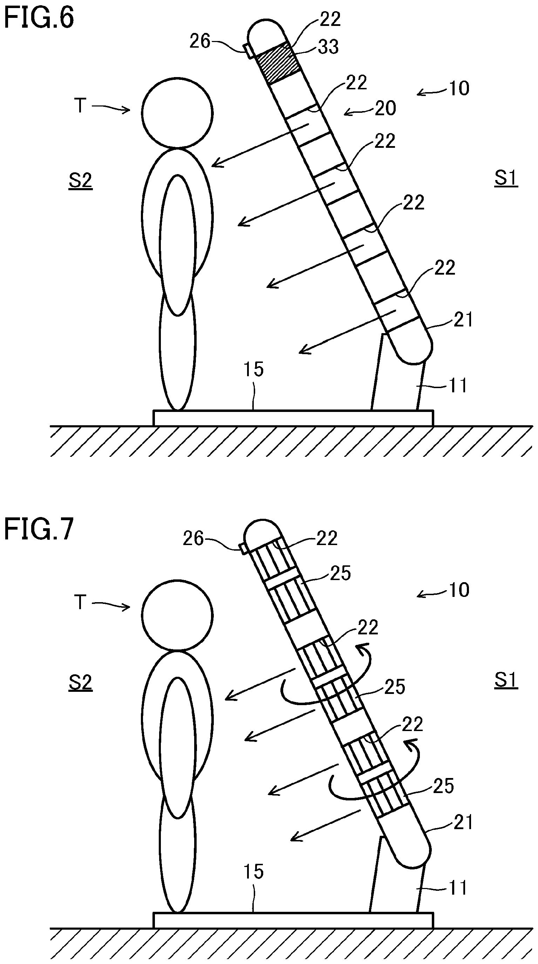

As illustrated in Fig. 6, the gate bodies (21) of the blower gates (20) each have five blow-out ports (22) that open so as to be spaced apart from one another in the vertical direction. The blow-out ports (22) are openable and closable by an opening-closing damper (33) (blow-out location changing part).

When the subject (T) passes between the blower gates (20), the height of the subject (T) is first detected by the detection sensor (26). On the basis of a result of the detection by the detection sensor (26), the position of the neck of the subject (T) is estimated.

The blow-out port (22) in a location where air is to be blown to the face of the subject (T) is closed by the opening-closing damper (33), thereby causing the air to be blown to the subject (T) from the neck down. In the example illustrated in Fig. 6, among the five blow-out ports (22), the first blow-out port (22) from the top is closed by the opening-closing damper (33), which is indicated by hatching.

### - Modification 2 of Embodiment 1 -

As illustrated in Fig. 7, the gate bodies (21) of the blower gates (20) each include three cross-flow type fans (25) disposed so as to be spaced apart from one another in the vertical direction.

When the subject (T) passes between the blower gates (20), the height of the subject (T) is first detected by the detection sensor (26). On the basis of a result of the detection by the detection sensor (26), the position of the neck of the subject (T) is estimated.

The operation of the fan (25) in a location where air is to be blown to the face of the subject (T) is stopped while the remaining fans (25) are operated, thereby causing the air to be blown to the subject (T) from the neck down. In the example illustrated in Fig. 7, among the three fans (25), the operation of the uppermost fan (25) is stopped.

### <<Embodiment 2>>

Embodiment 2 will be described.

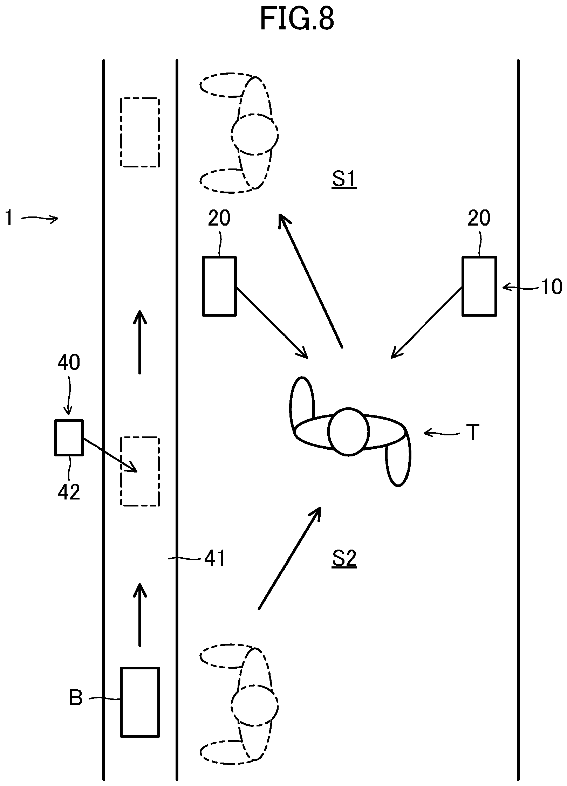

As illustrated in Fig. 8, a dust removal system (1) includes the dust removal device (10) for the subject (T), a conveyance part (41) that conveys an article (B) from the outdoor space (S2) to the indoor space (S1), and a dust removal device (40) for the article (B), such as bags, jackets, and the like.

As in Embodiment 1 mentioned above, the dust removal device (10) for the subject (T) includes the left-right pair of blower gates (20) and can remove the dust adhering to the body of the subject (T) by blowing air toward the subject (T), from the neck below, that passes between the blower gates (20).

The conveyance part (41) is constituted by a belt conveyer that extends from the outdoor space (S2) to the indoor space (S1), a lift that moves in the horizontal direction, and the like. The article (B) placed on the conveyance part (41) is conveyed toward the indoor space (S1).

The dust removal device (40) for the article (B) includes a fan (42) that blows air toward the article (B) during conveyance by the conveyance part (41). The dust removal device (40) for the article (B) is not limited to the fan (42) and may be constituted by, for example, a vibration mechanism that removes dust by applying vibrations to the article (B) during conveyance of the article (B).

In the thus configured dust removal system (1), the subject (T) first places the article (B), such as a hand bag and the like, on the conveyance part (41) and then moves to the dust removal device (10) for the subject (T). The article (B) is conveyed by the conveyance part (41) to the indoor space (S1), and, during the conveyance, dust is removed by the dust removal device (40) for the article (B).

The subject (T) passes through the dust removal device (10), and the dust adhering to the body is thereby removed. Thereafter, the subject (T) collects the article (B) that has been conveyed by the conveyance part (41) and enters the indoor space (S1).

Consequently, the dust adhering to the subject (T) and the dust adhering to the article (B) can be removed by different dust removal devices (10 and 40).

### Industrial Applicability

As described above, the present disclosure is useful for a dust removal device and a dust removal system. Reference Signs List

- 1: dust removal system
- 10: dust removal device
- 12: addition cartridge (addition part)
- 22: blow-out port
- 23: flap (blow-out location changing part)
- 25: fan
- 33: opening-closing damper (blow-out location changing part)
- 40: dust removal device
- S1: indoor space
- S2: outdoor space
- T: subject

## Claims

1. A dust removal device that blows air toward a subject (T) and removes dust, the dust removal device comprising:
a fan (25) that blows air;
a blow-out location changing part (23, 33) that changes a blow-out location of air that is blown by the fan (25) and moves toward the subject (T),wherein the blow-out location changing part (23, 33) is configured to change the blow-out location in accordance with a height of the subject (T) such that the air is blown toward the subject (T) from a neck down **characterised in that**
the blow-out location changing part (23) is configured to blow air toward the subject (T) in a direction from an indoor space (S1) to an outdoor space (S2), and blow-out ports (22) provided such that air is blown in a lateral direction that crosses an entrance direction in which the subject (T) moves from the outdoor space (S2) toward the indoor space (S1), wherein the blow-out port (22) that opens at a near side in the entrance direction is positioned higher than the blow-out port (22) that opens at a far side in the entrance direction.

2. The dust removal device according to claim 1,
wherein the blow-out location changing part (23) is configured to blow air toward the subject (T) downwardly from above.

3. The dust removal device according to any one of claims 1 to 2,
wherein the dust removal device includes an addition part (12) that adds a useful component to air that is blown by the fan (25).

4. A dust removal system comprising: the dust removal device (10) according to any one of claims 1 to 3, the dust removal device (10) being for the subject (T); and a dust removal device (40) that is for an article (B) and that blows air toward the article (B) and remove dust.

## Patentansprüche

1. Staubentfernungsvorrichtung, die Luft auf ein Subjekt (T) bläst und Staub entfernt, wobei die Staubentfernungsvorrichtung umfasst:
ein Gebläse (25), das Luft ausbläst;
einen Ausblasort-Änderungsteil (23, 33), der einen Ausblasort von Luft ändert, die von dem Gebläse (25) geblasen wird und sich zu dem Subjekt (T) bewegt, wobei der Ausblasort-Änderungsteil (23, 33) so konfiguriert ist, dass er den Ausblasort in Übereinstimmung mit einer Höhe des Subjekts (T) ändert, so dass die Luft von einem Hals abwärts zu dem Subjekt (T) geblasen wird,
**dadurch gekennzeichnet, dass**
der Ausblasort-Änderungsteil (23) so konfiguriert ist, dass er Luft in Richtung des Subjekts (T) in einer Richtung von einem Innenraum (S1) zu einem Außenraum (S2) bläst, und
Ausblasöffnungen (22) so vorgesehen sind, dass Luft in einer seitlichen Richtung geblasen wird, die eine Eintrittsrichtung kreuzt, in der sich das Subjekt (T) von dem Außenraum (S2) zu dem Innenraum (S1) bewegt, wobei die Ausblasöffnung (22), die sich an einer nahen Seite in der Eintrittsrichtung öffnet, höher angeordnet ist als die Ausblasöffnung (22), die sich an einer fernen Seite in der Eintrittsrichtung öffnet.

2. Staubentfernungsvorrichtung nach Anspruch 1,
wobei der Ausblasort-Änderungsteil (23) so konfiguriert ist, dass er Luft von oben nach unten in Richtung auf das Subjekt (T) bläst.

3. Staubentfernungsvorrichtung nach einem der Ansprüche 1 bis 2,
wobei die Staubentfernungsvorrichtung einen Zusatzteil (12) aufweist, der der vom Gebläse (25) eingeblasenen Luft eine nützliche Komponente hinzufügt.

4. Staubentfernungssystem, umfassend: die Staubentfernungsvorrichtung (10) nach einem der Ansprüche 1 bis 3, wobei die Staubentfernungsvorrichtung (10) für das Subjekt (T) ist; und eine Staubentfernungsvorrichtung (40), die für einen Gegenstand (B) ist und die Luft in Richtung des Gegenstands (B) bläst und Staub entfernt.

## Revendications

1. Dispositif de dépoussiérage soufflant de l'air vers un sujet (T) et dépoussiérant, le dispositif de dépoussiérage comprenant :
un ventilateur (25) soufflant de l'air ;
un élément de changement de lieu de soufflage (23, 33) changeant un lieu de soufflage de l'air soufflé par le ventilateur (25) et se déplaçant vers le sujet (T), l'élément de changement de lieu de soufflage (23, 33) étant configuré pour changer le lieu de soufflage en fonction de la hauteur du sujet (T) de sorte que l'air soit soufflé en direction du sujet (T) du cou vers le bas,
**caractérisé en ce que**
l'élément de changement de lieu de soufflage (23) est configuré pour souffler de l'air vers le sujet (T) dans une direction allant d'un espace intérieur (S1) à un espace extérieur (S2), et des orifices de soufflage (22) sont agencés de sorte que l'air soit soufflé dans une direction latérale croisant une direction d'entrée dans laquelle le sujet (T) se déplace de l'espace extérieur (S2) vers l'espace intérieur (S1), l'orifice de soufflage (22) s'ouvrant sur un côté proximal dans la direction de l'entrée étant positionné plus haut que l'orifice de soufflage (22) s'ouvrant sur un côté distal dans la direction de l'entrée.

2. Dispositif de dépoussiérage selon la revendication 1,
l'élément de changement de lieu de soufflage (23) étant configuré pour souffler de l'air en direction du sujet (T) du haut vers le bas.

3. Dispositif de dépoussiérage selon une quelconque des revendications 1 à 2,
le dispositif de dépoussiérage comprenant un élément additionnel (12) ajoutant un composant utile à l'air soufflé par le ventilateur (25).

4. Système de dépoussiérage comprenant : le dispositif de dépoussiérage (10) selon une quelconque des revendications 1 à 3, le dispositif de dépoussiérage (10) étant pour le sujet (T) ; et un dispositif de dépoussiérage (40), qui est pour un article (B), soufflant de l'air vers l'article (B) et dépoussiérant.
